# EUROPEAN PATENT APPLICATION

(11) **EP 3 100 724 A1**
(43) Date of publication of application: **07.12.2016**
(21) Application number: 14880497.4
(22) Date of filing: 22.10.2014
(51) Int. Cl.: A61K 31/122, A23L 33/10, A23L 2/52, A61K 35/74, A61P 27/02, A61P 27/06

(54) **MEDICINE FOR PREVENTING ISCHEMIC DISEASES**

(30) Priority: 30.01.2014 JP 2014016118
(71) Applicant: JX Nippon Oil & Energy Corporation, Chiyoda-ku Tokyo 100-8162 (JP)
(72) Inventor: HARA, Hideaki, Gifu-shi Gifu 501-1196 (JP); ISHIBASHI, Takashi, Tokyo 100-8162 (JP)
(74) Representative: Herzog, Fiesser & Partner Patentanwälte PartG mbB
(86) International application number: PCT/JP2014/078677
(87) International publication number: WO 2015/114900

(57) **Abstract**

The object of the present invention is to provide a prophylactic agent for retinal disorder such as ischemic retinal disorder. Specifically, the present invention relates to a prophylactic agent for retinal disorder, which contains, as an active ingredient, a carotenoid mixture containing astaxanthin as a main component.

## Description

### Technical Field

The present invention relates to a prophylactic agent for retinal disorder containing a carotenoid, for example.

### Background Art

In recent years, as the number of cases of lifestyle diseases has been increasing, the number of cases of atherosclerosis involving arterial thickening and stiffening has also been increasing. Risk factors such as hyperlipidemia, diabetes, hypertension, and smoking are considered to induce atherosclerosis in which atheromatous elevation is observed inside the arteries, resulting in vascular occlusion and stenosis. If vascular occlusion prevents oxygen and nutritional components from being transported, it may result in the onset of apoplexy, cerebral thrombosis, subarachnoid hemorrhage, myocardial infarction, amaurosis fugax, or the like. Further, if vascular stenosis or temporal occlusion prevents blood supply, it may cause the onset of cerebral ischemia, angina, ocular ischemia, or the like.

Hitherto, prevention of various diseases caused by arteriosclerosis has been awaited.

Meanwhile, it is known that carotenoids such as astaxanthin are widely distributed in animals, plants, and microorganisms and exhibit various types of action.

Nevertheless, it has been unknown that carotenoids such as astaxanthin have protective effects against ocular ischemia such as ischemic retinal disorder.

### Summary of Invention

In consideration of the above circumstances, the object of the present invention is to provide a prophylactic agent for retinal disorder such as ischemic retinal disorder.

As a result of intensive studies in order to achieve the above object, the present inventors found that a carotenoid mixture containing astaxanthin as a main component exhibits retinal protective action. This has led to the completion of the present invention.

The present invention encompasses the following.
(1) A prophylactic agent for retinal disorder, comprising, as an active ingredient, a carotenoid mixture containing astaxanthin as a main component.
(2) The prophylactic agent for retinal disorder according to (1), wherein the retinal disorder is ischemic retinal disorder.
(3) The prophylactic agent for retinal disorder according to (1) or (2), wherein the carotenoid mixture containing astaxanthin as a main component is derived from a microorganism belonging to the genus *Paracoccus.*
(4) The prophylactic agent for retinal disorder according to any one of (1) to (3), wherein the carotenoid mixture containing astaxanthin as a main component comprises 30% by mass to 70% by mass of astaxanthin, 4% by mass to 22% by mass of adonirubin, and 7% by mass to 13% by mass of adonixanthin, based on the total mass of the mixture.
(5) The prophylactic agent for retinal disorder according to any one of (1) to (4), which is administered so that the daily dose of the carotenoid mixture containing astaxanthin as a main component is 0.01 to 400 mg/kg body weight.
(6) The prophylactic agent for retinal disorder according to any one of (1) to (5), which is for improving a disease selected from the group consisting of diabetic retinopathy, glaucoma, ocular hypertension, and ocular ischemia.
(7) A pharmaceutical product, a functional food, a health food or a drink or a food comprising the prophylactic agent for retinal disorder according to any one of (1) to (6).

This description includes the content as disclosed in the description and/or drawings of Japanese Patent Application No. 2014-16118, which is a priority document of the present application.

### Brief Description of Drawings

Fig. 1 shows the test protocol used in Examples 1 and 2.
Fig. 2 shows the results of electroretinography (ERG) for ocular ischemia model mice fed with a carotenoid mixture containing astaxanthin as a main component.
Fig. 3 shows the results of histological evaluation of the retina for ocular ischemia model mice fed with a carotenoid mixture containing astaxanthin as a main component.
Fig. 4 shows the test protocol used in Examples 3 and 4.
Fig. 5 shows a graph indicating the cytoprotective action of a carotenoid mixture containing astaxanthin as a main component under OGD stress.
Fig. 6 shows a graph indicating the inhibitory effect of a carotenoid mixture containing astaxanthin as a main component upon reactive oxygen species (ROS) generation under OGD stress.

### Embodiments for Carrying Out the Invention

The present invention is described in detail below.

The prophylactic agent for retinal disorder of the present invention contains, as an active ingredient, a carotenoid mixture containing astaxanthin as a main component. By administering the prophylactic agent for retinal disorder of the present invention to humans, it is possible to prevent rental disorder and protect the retina from retinal disorder (especially ischemic retinal disorder) without side effects. This means that the prophylactic agent for retinal disorder of the present invention is also a retinal protective agent.

According to the present invention, a carotenoid mixture containing astaxanthin as a main component is used as an active ingredient. The term "carotenoid mixture containing astaxanthin as a main component" used herein refers to a carotenoid mixture containing astaxanthin, adonirubin, adonixanthin, canthaxanthin, echinenone, asteroidenone, 3-hydroxyechinenone, and β-carotene, in which the proportion accounted for by astaxanthin is greater than the proportions accounted for by other carotenoids. The carotenoid mixture containing astaxanthin as a main component may be a commercially available product or a product (such as a naturally occurring product) produced by a conventional chemical synthesis method, a fermentation method using a microorganism, or a method comprising extraction and purification from an animal, a plant, or the like. For example, a compound produced by extraction and purification from a microorganism (e.g., *Paracoccus carotinifaciens)* belonging to the genus *Paracoccus* can be used as the carotenoid mixture containing astaxanthin as a main component.

Specifically, in accordance with the method disclosed in JP Patent Publication (Kokai) No. 2009-50237 A, a carotenoid eluate is prepared by extraction from dry matter of a microorganism belonging to the genus *Paracoccus* with ethanol at 90°C to 100°C for 15 minutes or more. Residual bacterial cells are removed from the carotenoid eluate through filtration under heat (60°C to 70°C). The filtrate (extract) is cooled to 30°C and the degree of pressure reduction is adjusted to bring the container temperature to 30°C so as to concentrate the filtrate to one-fifth (1/15^{th}) of its original liquid volume. After concentration, the resulting product is heated at 60°C for 2 to 4 hours to re-isomerize astaxanthin in the cis form to astaxanthin in the trans form, followed by further aging at 30°C. After aging, a carotenoid mixture containing astaxanthin as a main component is collected by filtration. The carotenoid mixture containing astaxanthin as a main component is heated at 100°C under vacuum so as to be dried. For example, a carotenoid mixture extracted from a microorganism belonging to the genus *Paracoccus* (e.g., *Paracoccus* carotinifaciens) as described above contains carotenoids at the composition ratio shown in Table 1 below.

**Table 1**

| Component | Range of composition ratio for production (% by mass) |
|---|---|
| Astaxanthin | 47-67 |
| Adonirubin | 4-19 |
| Adonixanthin | 7-10 |
| Canthaxanthin | 2-6 |
| Echinenone | 1-2 |
| Asteroidenone | 0.5-2 |
| 3-Hydroxyechinenone | 0.5-1 |
| β-carotene | 0.5-1 |
| Lipid | 8-15 |
| Protein | 1-2 |
| Ash | 0.4-0.8 |
| Moisture | 0.2-0.4 |

Regarding the carotenoid mixture containing astaxanthin as a main component obtained by the production method in this embodiment, the lower limit of the astaxanthin content in the total mass of the carotenoid mixture is preferably 30% by mass or more, more preferably 35% by mass or more, and most preferably 40% by mass or more, and the upper limit thereof is preferably 70% by mass or less and more preferably 65% by mass or less. The lower limit of the adonirubin content in the total mass of the carotenoid mixture is preferably 4% by mass or more, more preferably 8% by mass or more, and most preferably 16% by mass or more, and the upper limit thereof is preferably 22% by mass or less and more preferably 20% by mass or less. The lower limit of the adonixanthin content in the total mass of the carotenoid mixture is preferably 7% by mass or more, more preferably 8% by mass or more, and most preferably 9% by mass or more, and the upper limit thereof is preferably 13% by mass or less and more preferably 12% by mass or less.

The prophylactic agent for retinal disorder of the present invention can be produced using, as an active ingredient, the carotenoid mixture containing astaxanthin as a main component described above.

The prophylactic agent for retinal disorder of the present invention may contain a pharmaceutically acceptable carrier (e.g., an excipient or a diluent) and additives which are appropriately selected from the group consisting of binders, bulking agents, lubricants, disintegrators, wetting agents, emulsifiers, buffering agents, suspending agents, preservatives, coloring agents, flavors, sweeteners, and the like, in addition to the carotenoid mixture containing astaxanthin as a main component. Such carrier and additives that can be used for producing the prophylactic agent for retinal disorder of the present invention are general products used for formulation preparation. Examples of binders include starch, polyvinylpyrrolidone, and hydroxypropyl methylcellulose. Examples of bulking agents include lactose and microcrystalline cellulose. Examples of lubricants include talc, silica, and magnesium stearate. Examples of disintegrators include starch and sodium starch glycolatestarch. Examples of wetting agents include sodium lauryl sulfate. Examples of emulsifiers include cellulose derivatives and sorbitol. In addition, examples of preservatives include methyl-p-hydroxybenzoate and sorbic acid. Note that additives that can be used in the present invention are not limited to the above examples.

The prophylactic agent for retinal disorder of the present invention can be formulated as a pharmaceutical product for oral or parenteral administration (e.g., intravenous, intra-arterial, intraperitoneal, transrectal, subcutaneous, intramuscular, sublingual, transnasal, or transvaginal administration). Examples of the dosage form include, but are not particularly limited to, liquid solutions, tablets, powders, granules, capsules, suppositories, sprays, controlled-release agents, suspensions, and drinks.

The dose of a carotenoid mixture containing astaxanthin as a main component that is contained in the prophylactic agent for retinal disorder of the present invention would vary depending on factors such as patient's age, body weight, gender, and conditions. For example, the daily dose of the prophylactic agent for retinal disorder of the present invention is, but is not limited to, 0.01 to 400 mg/kg body weight and preferably 0.1 to 40 mg/kg body weight in terms of the dose of the carotenoid mixture containing astaxanthin as a main component. Optionally, the daily dose may be divided into several doses, for example, 2 to 3 doses, for separate administration. In addition, the prophylactic agent for retinal disorder of the present invention may be administered in combination with a different prophylactic agent for retinal disorder to patients.

Further, due to the retinal disorder preventative effect of the prophylactic agent for retinal disorder of the present invention, the prophylactic agent for retinal disorder of the present invention can be used also for improving diseases such as diabetic retinopathy, glaucoma, ocular hypertension, ocular ischemia, and related diseases.

Furthermore, the prophylactic agent for retinal disorder of the present invention can be prepared in the form of a functional food, a health food or a drink or a food by adding or including the effective dose of the prophylactic agent for retinal disorder of the present invention in an arbitrary selected dosage form, such as in tablets, capsules, granules, drinks, and pet bottle beverages, or adding it to an arbitrary selected food or drink containing substantially no carotenoids. Examples of functional foods, health foods, drinks and foods include, but are not limited to, snacks, retort foods, juice products, teas, and dairy products. In addition, a sweetener, a seasoner, an emulsifier, a suspending agent, an antiseptic agent, and the like may optionally be added to functional foods, health foods, drinks and foods. Moreover, the prophylactic agent for retinal disorder of the present invention can also be used as a food additive.

Pharmacological evaluation of the prophylactic agent for retinal disorder of the present invention can be carried out *in vivo* using, for example, ocular ischemia model mice described in the Examples below. For example, the prophylactic agent for retinal disorder of the present invention is administered to mice and then the mice are treated to develop ocular ischemia. Upon electroretinography or histological evaluation of the retina, if the retina of mice treated with the prophylactic agent for retinal disorder of the present invention are in a state similar to that of the retina of normal mice, when compared with ocular ischemia model mice to which the prophylactic agent for retinal disorder of the present invention has not been administered, it can be judged that the prophylactic agent for retinal disorder of the present invention has retinal disorder preventing action (such as retinal protective action).

Alternatively, pharmacological evaluation of the prophylactic agent for retinal disorder of the present invention can be carried out *in vitro* using the retinal ganglion cell line (RGC-5 cell) described in the Examples below. For example, the retinal ganglion cell line is cultured in the presence of the prophylactic agent for retinal disorder of the present invention under OGD stress (corresponding to a state in which the supply of oxygen and glucose is restricted). OGD stress induces cell death and reactive oxygen species (ROS) generation. If cell death or reactive oxygen species (ROS) generation are found to be significantly inhibited based on a comparison with the retinal ganglion cell line exposed to OGD stress in the absence of the prophylactic agent for retinal disorder of the present invention, it can be judged that the prophylactic agent for retinal disorder of the present invention has retinal disorder preventing action (such as retinal protective action).

Meanwhile, the present invention also relates to a method for preventing retinal disorder (e.g., ischemic retinal disorder) or a method for improving a disease selected from the group consisting of diabetic retinopathy, glaucoma, ocular hypertension, and ocular ischemia comprising administering a carotenoid mixture containing astaxanthin as a main component to a patient or subject (i.e., a human) in accordance with the above description of the prophylactic agent for retinal disorder of the present invention.

The present invention is hereinafter described in more detail with reference to Examples. However, the technical scope of the present invention is not limited to the Examples.

The materials and method used in Examples 1 and 2 are described below.

### (1) Production of the carotenoid mixture containing astaxanthin as a main component

In accordance with the method disclosed in JP Patent Publication (Kokai) No. 2009-50237 A, an carotenoid eluate was prepared by extraction from dry matter of a microorganism belonging to the genus *Paracoccus* with ethanol at 90°C for 20 minutes. Residual bacterial cells were removed from the carotenoid eluate through filtration under heat at 65°C. The filtrate (extract) was cooled to 30°C and the degree of pressure reduction was adjusted to bring the container temperature to 30°C so as to concentrate the filtrate to one-fifth (1/5^{th}) of its original liquid volume. After concentration, the resulting product was heated at 60°C for 3 hours to re-isomerize astaxanthin in the cis form to astaxanthin in the trans form, followed by further aging at 30°C for 12 hours. After aging, a carotenoid mixture containing astaxanthin as a main component was collected by filtration. The carotenoid mixture containing astaxanthin as a main component was heated at 100°C in a vacuum so as to be dried. The composition of the obtained carotenoid mixture containing astaxanthin as a main component is shown in Table 2 below. In addition, the obtained carotenoid mixture containing astaxanthin as a main component is hereinafter referred to as "astaxanthin for health foods."

**Table 2**

| Component | Composition ratio (% by mass) |
|---|---|
| Astaxanthin | 47.5 |
| Adonirubin | 18.3 |
| Adonixanthin | 8.0 |
| Canthaxanthin | 5.1 |
| Echinenone | 1.6 |
| Asteroidenone | 1.3 |
| 3-Hydroxyechinenone | 1.0 |
| β-carotene | 0.8 |
| Lipid | 14.0 |
| Protein | 1.5 |
| Ash | 0.6 |
| Moisture | 0.3 |
| Total | 100.0 |

### (2) Preparation of ocular ischemia model mice

Ocular ischemia model mice were prepared by ligating the pterygopalatine artery (PPA) and the external carotid artery (ECA) in accordance with the method disclosed in OGISHIMA H. et al., Ligation of the Pterygopalatine and External Carotid Arteries Induces Ischemic Damage in the Murine Retina, Investigative Ophthalmology & Visual Science, 2011, Vol. 52, No. 13, pp. 9710-9720.

### (3) Test method

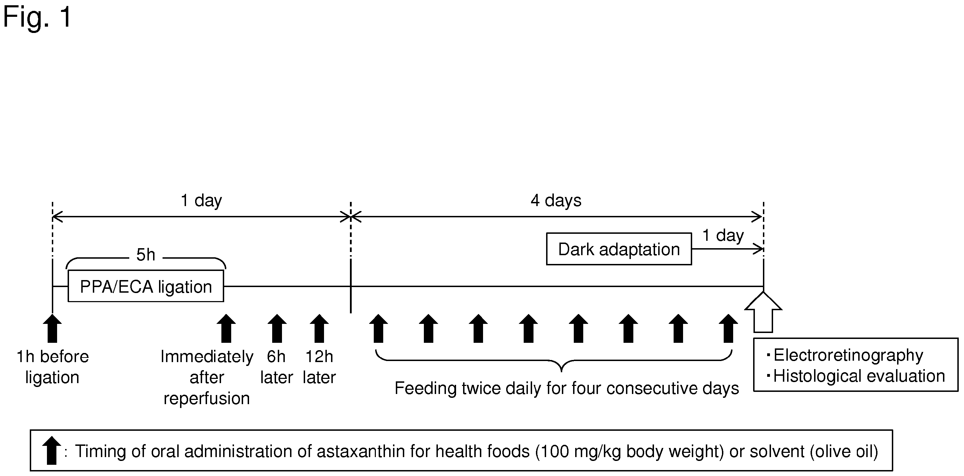

As shown in Fig. 1, ligation of PPA and ECA was performed on each ocular ischemia model mouse to induce ischemia for 5 hours, followed by reperfusion. Astaxanthin for health foods was suspended in olive oil, and ocular ischemia model mice were fed therewith at a dose of 100 mg/kg body weight. The mice were fed 12 times in total (1 hour before PPA/ECA ligation, immediately after reperfusion, 6 hours and 12 hours after reperfusion, twice daily from the day following ligation to 4 days later) via oral administration.

The mice were subjected to dark adaptation from day 4 after ischemic reperfusion. On day 5, electroretinography (ERG) was performed for measurement, and paraffin sections of eyeballs were prepared to carry out histological evaluation using hematoxylin and eosin staining in accordance with the method disclosed in OGISHIMA H. et al., Ligation of the Pterygopalatine and External Carotid Arteries Induces Ischemic Damage in the Murine Retina, Investigative Ophthalmology & Visual Science, 2011, Vol. 52, No. 13, pp. 9710-9720.

In addition, ocular ischemia model mice were fed with a solvent (olive oil) to create a group of non-administration of astaxanthin for health foods. Each of the experimental groups consisted of 10 mice, which were 3 groups, i.e., an unligated group, a group of administration of astaxanthin for health foods + ischemic reperfusion, and a group of solvent administration + ischemic reperfusion.

### [Example 1] Electroretinography (ERG) for measurement

Electroretinography is a measurement involving collectively obtaining electric potentials corresponding to electrical activity in sensory retina and retinal pigment epithelium induced by light stimulation in a wide area of the ocular fundus. An a-WAVE represents a corneal-negative electric potential deflection, which is observed at a latent time of 1.5 msec following flush stimulation to eyeballs. An a-WAVE is considered to correspond to electric potential originating from the retinal surface layer (stratum neuroepitheliale retinae). The clinical meaning of an abnormal a-WAVE is ophthalmic artery obstruction, retinal detachment, or the like. Meanwhile, a b-WAVE represents a significant corneal-positive electric potential deflection, which is observed after an a-WAVE. A b-WAVE is considered to correspond to electric potential originating from Muller cells (ranging from the interlayer to the lining) of the retina. The clinical meaning of a small b-WAVE amplitude is suspected occlusion of the central retinal artery, congenital retinoschisis, congenital stationary night blindness, or the like.

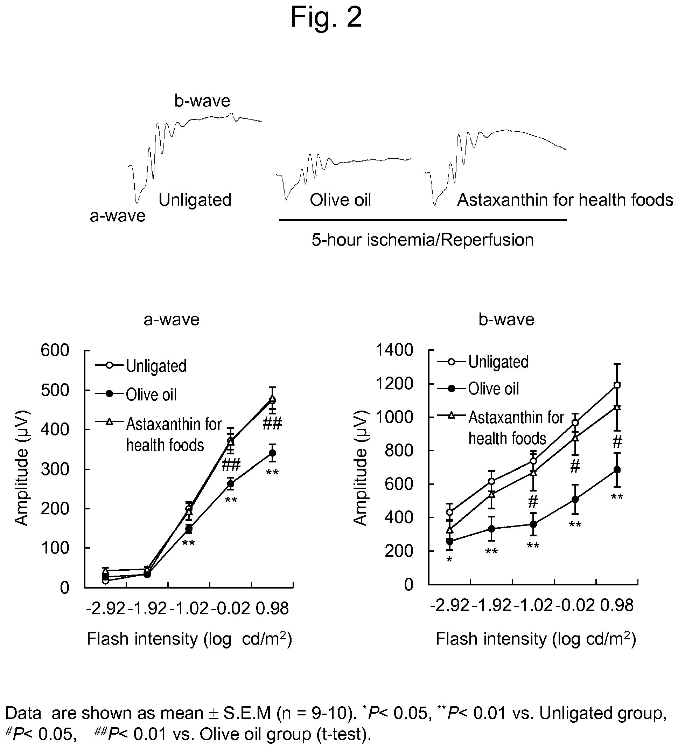

In this Example, as shown in Fig. 2, an a-WAVE and a b-WAVE were observed for ocular ischemia model mice fed with astaxanthin for health foods, which were similar to those observed for the unligated group (control). Electric potentials corresponding to an a-WAVE and a b-WAVE significantly decreased for the ocular ischemia model mice, compared with the ocular ischemia model mice fed with a solvent (olive oil) alone. It was therefore revealed that astaxanthin for health foods has a retinal protective effect. Accordingly, it is presumed that astaxanthin for health foods can also prevent the development of cerebral ischemia, angina, ocular ischemia, and the like.

### [Example 2] Histological evaluation of retina

Electroretinography (ERG) was performed for measurement, and paraffin sections of eyeballs were prepared to carry out histological evaluation using hematoxylin and eosin staining.

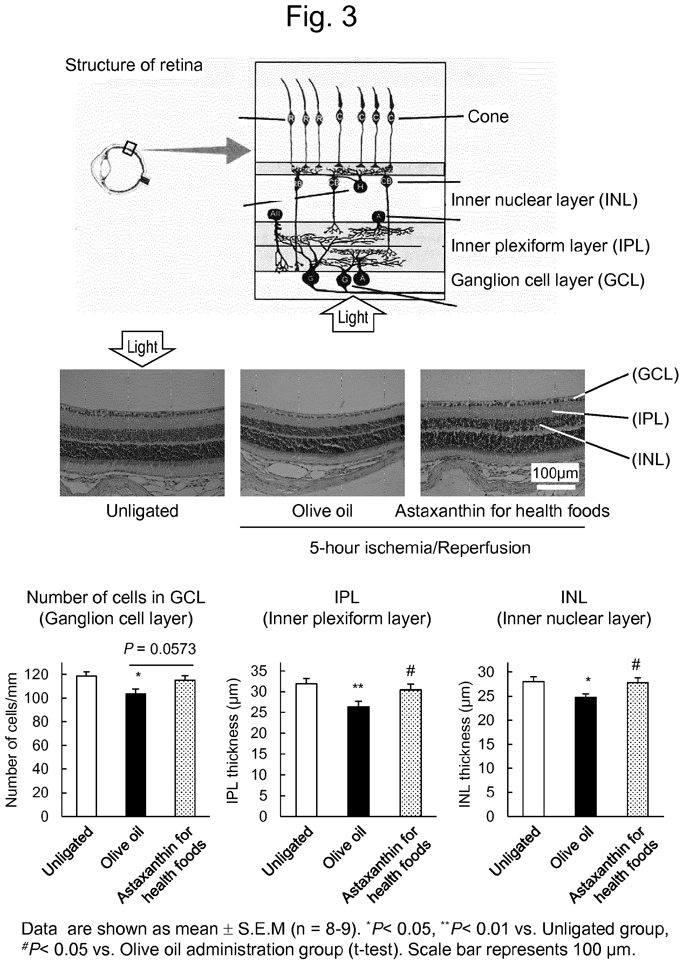

In this Example, as shown in Fig. 3, the retinal structure of the ocular ischemia model mice fed with astaxanthin for health foods was found to be normal, as in the case of the non-ligated mice. However, the retinal structure of the ocular ischemia model mice fed with a solvent alone was found to have been disturbed.

In addition, a comparison of the number of cells in the ganglion cell layer (GCL) of the retina showed that the number of cells for the group fed with astaxanthin for health foods was significantly greater than that for the group fed with a solvent alone and comparable to the number of cells for the unligated group.

Further, a comparison of the thickness of the inner plexiform layer (IPL) and the thickness of the inner nuclear layer (INL) showed that the cell thickness for the group fed with astaxanthin for health foods was significantly greater than that for the group fed with a solvent alone and comparable to the cell thickness for the unligated group.

The above results revealed that astaxanthin for health foods has a retinal protective effect. Accordingly, it is presumed that astaxanthin for health foods can also prevent the development of cerebral ischemia, angina, ocular ischemia, and the like because it can prevent damage to cells with poor blood flow supply.

The materials and method used in Examples 3 and 4 are described below.

### (1) RGC-5 cell line

The RGC-5 cell line is a cell line from rat retinal ganglion cells and used for *in vitro* tests for cell damage evaluation. The RGC-5 cell line can be cultured in accordance with the method disclosed in NAKAJIMA Y. et al., Zeaxanthin, a Retinal Carotenoid, Protects Retinal Cells against Oxidative Stress, Current Eye Research, 2009, Vol. 34, pp. 311-318.

### (2) Test method

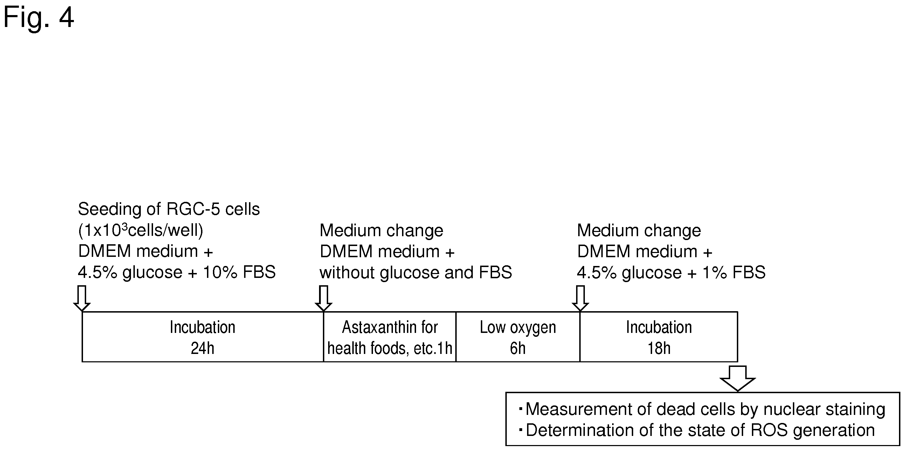

As shown in Fig. 4, the RGC-5 cell line was cultured under OGD stress in order to reproduce conditions of poor blood flow. Here, the expression OGD stress refers to a state in which the supply of oxygen and glucose is restricted.

RGC-5 cells were added at a concentration of 1 × 10³ cells/well to a DMEM medium containing glucose (4.5%) that was concentrated at 4.5 times the general concentration and fetal bovine serum (FBS: 10%) of Oseanian origin that was concentrated at 10 times the general concentration, followed by culture for 24 hours.

Next, the mixture was washed twice with FBS-free base medium. Then, glucose and FBS were removed. Astaxanthin for health foods described in Examples 1 and 2, a solvent (DMSO), and Edaravone (a free radical scavenger that is a pharmaceutical product) were separately added, followed by culture for 1 hour.

Thereafter, aeration conditions (air 95% + CO₂ 5%) were changed to low-oxygen conditions (N₂ 94% + O₂ 1% + CO₂ 5%), followed by further culture for 6 hours.

Then, the mixture was washed twice with FBS-free base medium, followed by culture in DMEM medium containing high-concentration glucose (4.5%) and general-concentration FBS (1%) for 18 hours. The dead cell rate and the state of reactive oxygen species (ROS) generation were determined via nuclear staining. Nuclear staining was carried out in accordance with the method disclosed in OTSUKA T. et al., The Protective Effects of a Dietary Carotenoid, Astaxanthin, Against Light-Induced Retinal Damage, Journal of Pharmacological Sciences, 2013, Vol. 123, No. 3, pp. 209-218. Meanwhile, the state of reactive oxygen species (ROS) generation was determined in accordance with the method disclosed in NAKAJIMA Y. et al., Zeaxanthin, a Retinal Carotenoid, Protects Retinal Cells against Oxidative Stress, Current Eye Research, 2009, Vol. 34, pp. 311-318.

### [Example 3] Cytoprotective action under OGD stress

In nuclear staining, only dead cells (PI-positive cells) are stained.

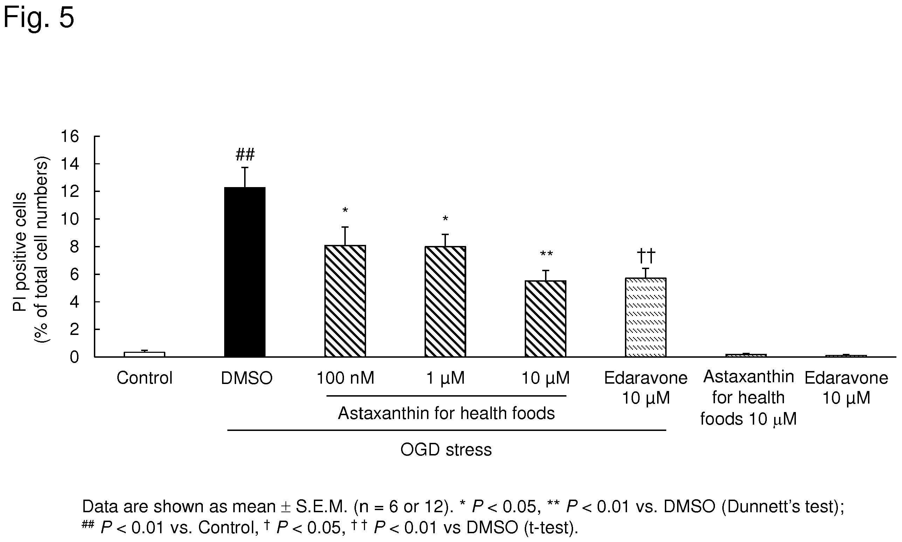

As shown in Fig. 5, the number of dead RGC-5 cells supplemented with astaxanthin for health foods was significantly lower than the number of dead RGC-5 cells supplemented with a solvent (DMSO). In the case in which 10 µM of astaxanthin for health foods had been added, the cytoprotective effect was comparable to that in the case in which there had been addition of the pharmaceutical product, i.e., Edaravone.

Further, astaxanthin for health foods and Edaravone did not cause cell death in the absence of OGD stress.

### [Example 4] Inhibitory effect on reactive oxygen species (ROS) generation under OGD stress

The expression reactive oxygen species (ROS) refers to oxygen molecules that are in a more activated state than that of usual oxygen molecules, and related substances thereof.

It is known that ROS (hydroxyl radical or singlet oxygen) causes oxidation and denaturation of unsaturated fatty acid, which induces damage to vascular endothelial cells and then arteriosclerosis, thereby resulting in a predisposition for the likely development of vascular stenosis and thrombus. That is, if ROS generation can be inhibited, arteriosclerosis and the like can be prevented.

In this Example, poor blood flow conditions were reproduced with the application of OGD stress to examine the inhibitory effect of astaxanthin for health foods on ROS generation.

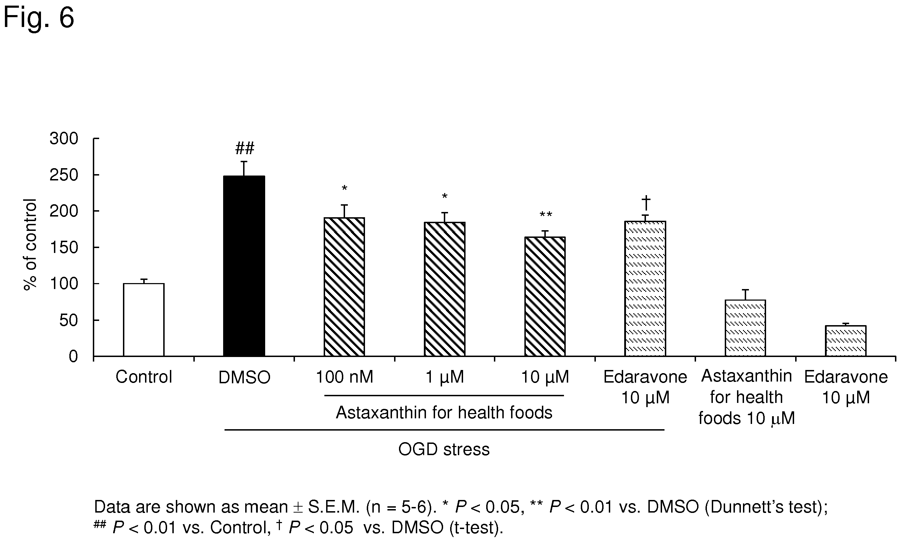

As shown in Fig. 6, ROS production of RGC-5 cells supplemented with astaxanthin for health foods was significantly lower than that of RGC-5 cells supplemented with a solvent (DMSO). Astaxanthin for health foods inhibited ROS generation at a concentration lower than that of the pharmaceutical product, i.e., Edaravone.

In the case in which there had been no application of OGD stress, astaxanthin for health foods did not exhibit the inhibitory effect on ROS generation.

In this Example, it was found that astaxanthin for health foods has a retinal protective effect under OGD stress.

It is presumed that astaxanthin for health foods can also prevent the development of cerebral ischemia, angina, ocular ischemia, and the like because it can inhibit the generation of ROS that causes damage to cells.

### Industrial Applicability

According to the present invention, a prophylactic agent for retinal disorder that is useful for eye health is provided.

All publications, patents, and patent applications cited herein are incorporated herein by reference in their entirety.

## Claims

1. A prophylactic agent for retinal disorder, comprising, as an active ingredient, a carotenoid mixture containing astaxanthin as a main component.

2. The prophylactic agent for retinal disorder according to claim 1, wherein the retinal disorder is ischemic retinal disorder.

3. The prophylactic agent for retinal disorder according to claim 1 or 2, wherein the carotenoid mixture containing astaxanthin as a main component is derived from a microorganism belonging to the genus *Paracoccus.*

4. The prophylactic agent for retinal disorder according to any one of claims 1 to 3, wherein the carotenoid mixture containing astaxanthin as a main component comprises 30% by mass to 70% by mass of astaxanthin, 4% by mass to 22% by mass of adonirubin, and 7% by mass to 13% by mass of adonixanthin, based on the total mass of the mixture.

5. The prophylactic agent for retinal disorder according to any one of claims 1 to 4, which is administered so that the daily dose of the carotenoid mixture containing astaxanthin as a main component is 0.01 to 400 mg/kg body weight.

6. The prophylactic agent for retinal disorder according to any one of claims 1 to 5, which is for improving a disease selected from the group consisting of diabetic retinopathy, glaucoma, ocular hypertension, and ocular ischemia.

7. A pharmaceutical product, a functional food, a health food or a drink or a food comprising the prophylactic agent for retinal disorder according to any one of claims 1 to 6.
